# EUROPEAN PATENT APPLICATION

(11) **EP 1 754 443 A1**
(43) Date of publication of application: **21.02.2007**
(21) Application number: 04746035.7
(22) Date of filing: 10.06.2004
(51) Int. Cl.: A61B 10/00

(54) **LIVING BODY INFORMATION MEASURING INSTRUMENT, STANDARD ELEMENT, AND METHOD OF USING LIVING BODY INFORMATION MEASURING INSTRUMENT**

(30) Priority: 10.06.2004 JP 2004172101
(71) Applicant: Matsushita Electric Industrial Co., Ltd., Kadoma-shi Osaka 571-8501 (JP)
(72) Inventor: UCHIDA, Shinji, .. (JP)
(74) Representative: Grünecker, Kinkeldey, Stockmair & Schwanhäusser Anwaltssozietät
(86) International application number: PCT/JP2004/008520
(87) International publication number: WO 2005/120361

(57) **Abstract**

When there is any abnormality or characteristic change in the light intensity of a light source or the sensitivity of a light detector, measurement accuracy decreases.

An apparatus of the present invention comprising: a light source 11; a living body measuring optical element 12 for applying light emitted from the light source 11 to a living body and receiving light returning from the living body; a light detector 16 for detecting the light received by the living body measuring optical element 12; and a light guide 14 which can be arranged in contact with the living body measuring optical element 12, in which an abnormality is detected or corrected by use of output from the light detector 16 in a state the light guide 14 is arranged in contact with the living body measuring optical element 12.

## Description

### Technical Field

The present invention relates to a biological information measuring apparatus and a reference element for use in the non-invasive measurement of glucose, cholesterol, urea, triglyceride and so forth in body fluids based on optical measurement of living tissues, and also to a method of using the biological information measuring apparatus.

### Background Art

Conventionally, various biological information measuring apparatuses and calibration methods have been proposed for measuring specific components in a living body or a solution.

For example, according to Japanese Patent No. 2648377, biological information within a living body is measured through the use of near-infrared light by emit light inside a living body from a light source disposed on the living-body surface and receiving, light which returns again onto the surface of the living body after propagating through the living body as being scattered and absorbed in the living body.

In this case, however, there is such a problem that, when the performance of the light source or detector changes with time, a measurement value varies and measurement accuracy decreases.

To cope with this, the amount of light is measured by applying light emitted from the light source to a reflection body arranged on a protection cover facing the light source, and receiving the reflected light by a light-receiving section arranged in the same plane as the light source. The obtained value is used as a calibration value for correcting measurement values.

However, the conventional optical measurement methods and optical measurement apparatuses as mentioned above have the following problems.

In the conventional methods, light emitted from a light source is applied to a reflection board attached to a protection cover and the light reflected from the reflection board is detected by a light-receiving section provided in the same plane as the light source.

Accordingly, such a method effectively works when the light source and the light receiving section are arranged in the same plane and the reflection board is arranged at a position facing them. Whereas, when a light-emitting section which emit light toward a living body and a light incident section which receive light from the living body are not arranged in the same plane, it is very difficult to apply light to the reflection board and receive the light reflected from the reflection board.

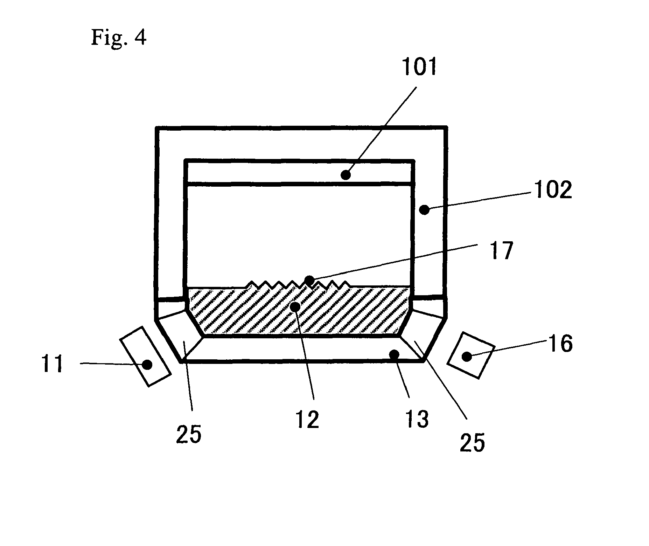

Figure 4 shows an optical measurement apparatus equipped with a reflection board. As shown in the figure, the optical measurement apparatus of Figure 4 is constituted of a light source 11, a living body measuring optical element 12, a holding table 13 and a light guide detection means 16. In addition, a reflection board 101 is provided on the opposite side to the opening portion of a cover 102.

The holding table 13 is a table for holding the living body measuring optical element 12. The holding table 13 has holes 25 for allowing light emitted from the light source 11 to strike the living body measuring optical element 12 and for allowing the light from the living body measuring optical element 12 to strike the light detector 16, respectively. A groovy portion 17 is provided to the living body measuring optical element 12. The groovy portion 17 has a light-emitting section for emitting light toward a living body and a light incident section for receiving light from the living body. These two sections are not arranged in the same plane and constitute a groovy form such that light emitted from the light emitting section can directly enter the light incident section. Therefore, it is difficult to apply light emitted from the light emitting section to the reflection board 101 and cause the reflected light to be received by the light incident section of the groovy portion 17. Thus, light emitted from the light source 11 cannot pass through the living body measuring optical element 12 again, with the result that the light cannot be detected by the light guide detection means 16.

### Disclosure of the Invention

The present invention was attained with a view to overcoming the aforementioned problems. An object of the present invention is to provide a biological information measuring apparatus and a reference element capable of detecting and correcting an abnormality, even if a light emitting section which emits light toward a living body and a light incident portion which receives light from the living body are not present in the same plane, and also to provide a method of using the biological information measuring apparatus.

In order to solve the aforementioned problems, the 1^{st} aspect of the present invention is a biological information measuring apparatus comprising:
a light source;
a living body measuring optical element of applying light emitted from said light source to a living body and receiving light returning from said living body;
a light detector of detecting said light received by said living body measuring optical element; and
a reference light guide capable of guiding the light applied by said living body measuring optical element so that the light can be returned to said living body measuring optical element, in a state of being arranged in contact with said living body measuring optical element.

The 2^{nd} aspect of the present invention is the biological information measuring apparatus according to the 1^{st} aspect of the present invention, further comprising a calculation section of providing biological information of said living body through calculation based on the light returned from said living body and detected by said light detector,
wherein the calculation section detects that at least one of said light source, said living body measuring optical element and said light detector abnormally functions based on the light detected by said light detector in a state where said light guide is arranged in contact with said living body measuring optical element.

The 3^{rd} aspect of the present invention is the biological information measuring apparatus according to the 1^{st} aspect of the present invention, further comprising a calculation section of providing biological information of said living body through calculation based on the light returning from said living body and detected by said light detector,
wherein the calculation section corrects said biological information based on the light detected by said light detector in a state where said light guide is arranged in contact with said living body measuring optical element.

The 4^{th} aspect of the present invention is the biological information measuring apparatus according to the 1^{st} aspect of the present invention, wherein a concave-convex portion is formed on a part of the surface of said living body measuring optical element, and the part of said light guide which is to be in contact with said living body measuring optical element is deformable.

The 5^{th} aspect of the present invention is the biological information measuring apparatus according to the 1^{st} aspect of the present invention, wherein said light guide is formed of a material having a refractive index higher than that of the air and lower than that of said living body measuring optical element.

The 6^{th} aspect of the present invention is the biological information measuring apparatus according to the 1^{st} aspect of the present invention, wherein said light guide is a scattering body.

The 7^{th} aspect of the present invention is the biological information measuring apparatus according to the 4^{th} aspect of the present invention, wherein said light guide is an elastic substance.

The 8^{th} aspect of the present invention is the biological information measuring apparatus according to the 7^{th} aspect of the present invention, wherein said light guide has an elasticity modulus of 1 to 10 MPa.

The 9^{th} aspect of the present invention is a reference element for use in a biological information measuring apparatus comprising:
a light source;
a living body measuring optical element of applying light emitted from said light source to a living body and receiving light returning from said living body; and
a light detector of detecting the light received by said living body measuring optical element,
the reference element comprising:
   a light guide capable of guiding the light applied by said living body measuring optical element so that it returns to said living body measuring optical element in a state of being arranged in contact with said living body measuring optical element.

The 10^{th} aspect of the present invention is the reference element according to the 9^{th} aspect of the present invention, further comprising a cover of covering a portion of said light guide other than the portion which is in contact with said living body measuring optical element.

The 11^{th} aspect of the present invention is the reference element according to the 9^{th} aspect of the present invention, wherein
a part of said light guide which is in contact with said living body measuring optical element is deformable.

The 12^{th} aspect of the present invention is the reference element according to the 9^{th} aspect of the present invention, wherein said light guide is formed of a material having a refractive index higher than that of the air and lower than that of said living body measuring optical element.

The 13^{th} aspect of the present invention is the reference element according to the 9^{th} aspect of the present invention, wherein said light guide is a scattering body.

The 14^{th} aspect of the present invention is the reference element according to the 11^{th} aspect of the present invention, wherein said light guide is an elastic substance.

The 15^{th} aspect of the present invention is the reference element according to the 14^{th} aspect of the present invention, wherein said light guide has an elasticity modulus of 1 to 10 MPa.

The 16^{th} aspect of the present invention is a method of using a biological information measuring apparatus using the biological information measuring apparatus according to the 1^{st} aspect of the present invention, comprising:
a biological information measuring step of measuring biological information based on the light detected by said light detector in a state where said living body measuring optical element is in contact with a target living body to be measured; and
an abnormality-correcting step of detecting or correcting an abnormality based on the light detected by said light detector in a state that said reference light guide is in contact with said living body measuring optical element.

The present invention employs a reference element having a light guide capable of guiding light applied by a living body measuring optical element so that the light can be returned to the living body measuring optical element in the state that the light guide is arranged in contact with the living body measuring optical element and also a living body measuring apparatus having the light guide. By using such element and apparatus, according to the present invention, the sensitivities of a light source, a living body measuring optical element and a light detector can be checked, and measurement errors caused by temperature change and secular change can be corrected.

### Brief Description of the Drawings

Figure 1 is a schematic view of a biological information measuring apparatus according to an embodiment of the present invention, in which a calibration element is not in contact with a living body measuring optical element;
Figure 2 is a schematic view of the biological information measuring apparatus according to an embodiment of the present invention, in which the calibration element is in contact with a living body measuring optical element;
Figure 3 is a view showing another structure of the living body measuring optical element according to an embodiment of the present invention; and
Figure 4 is a view showing that it is difficult to receive reflected light in an optical measurement apparatus equipped with a reflection board.

### (Description of Symbols)

- 11: Light source
- 12: Living body measuring optical element
- 13: Holding table
- 14: Light guide
- 15: Cover
- 16: Light detecting section
- 17: Groovy portion
- 21: Calculation section
- 22: Memory means
- 23: Display section

### Best Mode for Carrying Out the Invention

An embodiment of the present invention will be described below with reference to the drawings. The embodiment is described by way of an example. It should not be construed that the embodiment of the present invention is limited to the example.

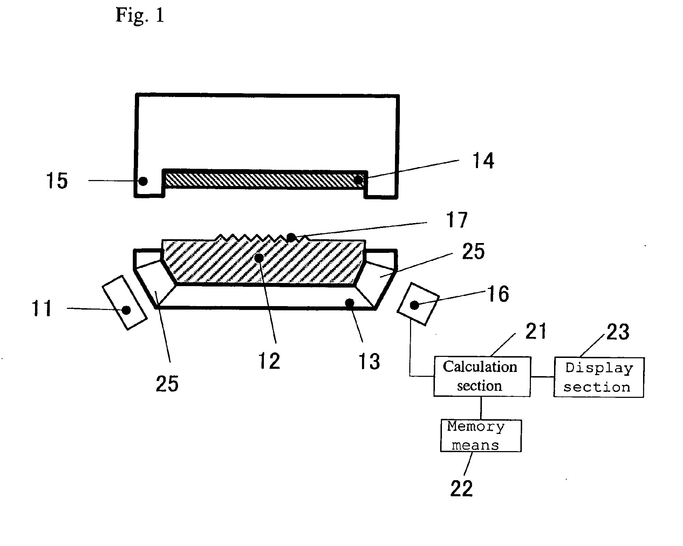

Figure 1 is a schematic view of a biological information measuring apparatus of the present invention.

As shown in Figure 1, the biological information measuring apparatus according to the embodiment is constituted of a light source 11, a living body measuring optical element 12, a holding table 13, a light guide 14, a cover 15, a light detecting means 16, a calculation section 21, a memory means 22, and a display section 23. The light guide 14 and the cover 15 constitute a calibration element. The calibration element according to this embodiment is an example of a reference element of the present invention.

The biological information measuring apparatus according to this embodiment is an apparatus of measuring information about the tissue near the surface of a living body. Specifically, the biological information measuring apparatus according to the embodiment has a groovy portion 17 at the surface portion of the living body measuring optical element 12 to be in contact with the surface of a living body, for measuring light passed through the tissue near the surface of a living body. The biological information measuring apparatus according to the embodiment is an apparatus of measuring light passed though a surface portion of a living body which is trapped by the groovy portion 17 by bringing the surface of the living body measuring optical element 12 into contact with the surface of the living body.

As the light source 11, any light source can be used as long as it has light having a wavelength absorbed by a target component to be measured.

For example, in the case of light within the middle-infrared region, a Globar light source having a sintered SiC rod, CO₂ laser, tungsten light, infraredpulse light source, and QCL light source may be used.

For the measurement of a substance such as a glucose which has a strong absorption peak in the middle-infrared region, e.g. , at a wavelength of 1033 cm⁻¹ or 1080 cm⁻¹, a Glober light source, infrared pulse light source, and QCL light source may be preferably used although a light source employed is not particularly limited to them.

For the measurement of a substance having an absorption wavelength in a near-infrared region, for example, a halogen light source, semiconductor laser, or LED may be used.

It is known that glucose has an absorption peak not only in the middle-infrared region but also in the near-infrared region. Therefore, use of LED may be particularly preferable.

As a material for the living body measurement optical element 12, materials known in the art can be used.

For example, for measuring a substance having an absorption wavelength in the middle-infrared region, silicon, germanium, SiC, diamond, ZnSe, ZnS, or Krs may be used.

For measuring a substance such as glucose which exhibits an absorption peak in the middle-infrared region, e.g., a wavelength of 1033 cm⁻¹ or 1080 cm⁻¹, silicon or germanium is particularly preferable from the view point of not only high permeability at an infrared wavelength of about 9 to 10 microns but also high processability and mechanical strength.

When a substance that absorbs light at a wavelength within the near-infrared region is to be measured, fused quartz, single crystalline silicon, optical glass, plastic, or transparent resin is used.

As for the shape of the groovy portion 17 formed on the living body measuring optical element 12, a V-shaped groove as shown in the figure may be employed. However, the shape is not limited to this type. A U-shaped or a step-form groove may be used.

Reference numeral 13 denotes a holding table for holding a living body measurement optical element 12. The holding table 13 has hole portions 25, though which light emitted from the light source 11 strikes the living body measuring optical element 12 and the light derived from the living body measurement optical element 12 strikes the light detector 16.

Reference numeral 14 is a light guide for correcting a detection signal of light amount to be detected by the optical detector 16. Reference numeral 15 is a cover for holding the light guide 14. Note that the light guide 14 and the cover 15 may be detachably attached to the main body having the living body measurement optical element 12. Alternatively, the light guide 14 and the cover 15 may be fixed to the main body having the living body measurement optical element 12.

As the light guide 14, a deformable material is used having a refraction index higher than that of the air and smaller than that of the living body measurement optical element.

A material for the light guide 14 is preferably an elastic material like rubbers such as acrylic rubber, urethane rubber, silicone rubber, and fluorine rubber. Use of such an elastic material as a material for the light guide 14 has an advantage that the light guide 14 can be repeatedly used for calibration.

An elastic material more preferably has an elastic modulus of 1 to 10 MPa. This is because it can easily contact with the living body measurement optical element.

Furthermore, a rubber for general purposes, such as styrene-butadiene rubber, butadiene rubber, and isoprene rubber, or other type of rubber such as nitrile rubber, chloroprene rubber, and butyl rubber, may be used.

In particular, silicone rubber may be useful since it has a virtually inactive property to the living body, as seen in frequent uses, as an artificial prosthesis for mary prosthesis, artificial ear, and artificial nose.

Furthermore, it may also be preferable to use an elastomer for medical purposes, such as polyethylene glycol, polypropylene glycol, and polytetramethylene glycol.

Moreover, it may also be preferable to use a hydrogel, which is formed by introducing a crosslinking structure, a hydrophobic group, or a crystal structure into a water-soluble polymer and swelling the obtained polymer with water. The hydrogel can be said to be preferable since it is not only soft and harmless to the tissue but also highly permeable to a substance. For example, polyhydroxyethyl methacrylate, polyacrylamide, and polyvinylpyrrolidone are preferable.

Absorption coefficient of the light guide is not particularly limited. It may be better to select a light guide having a smaller absorption coefficient within the range of wavelength to be used.

As the light guide 14, a scattering body may be used. As a scattering body, resins of high-density polyethylene and polycarbonate or the like are preferably used. In particular, a light-diffusion grade of polycarbonate is useful since its diffused light beam is highly permeable.

As the light detecting means 16, items known in the art can be used. For example, for the middle-infrared region, a pyroelectric sensor, a thermopile, a thermister, and an MCT detector (HgCdTe detector as a quantum detector) may be used. For the near-infrared region , an InGaAs detector, aphotodiode, a PbS detector, an InSb detector, and an InAs detector, may be exemplified. Based on a signal (not shown) detected by the light detecting means 16, the calculation section 21 calculates and provides a parameter of a living tissue, such as a glucose concentration. The display section 23 displays the results of the calculation performed in the calculation section 21. As the display section 23, a liquid crystal display and an EL display may be used. When the result is output by voice in the display section 23, a speaker may be used. The calculation section 21 is constituted of a CPU and a memory.

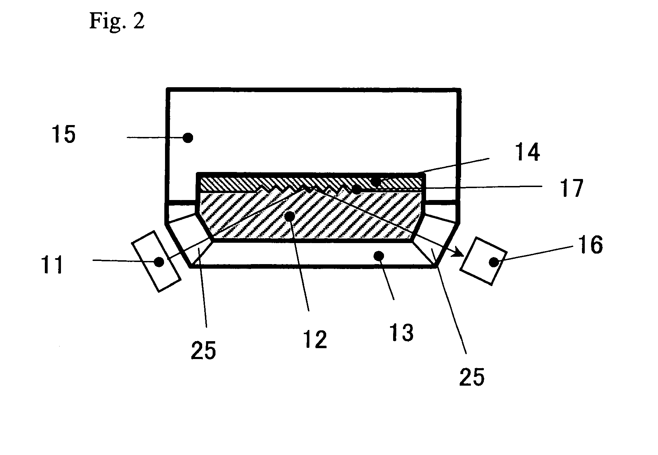

Next, a calibration method using a biological information measuring apparatus according to this embodiment will be explained with reference to Figures 1 and 2.

As shown in Figure 1, the light guide 14 is pressed against the living body measuring element 12 by holding the cover 15 having the light guide 14. In this manner, the light guide 14 can be brought into contact with the groovy portion 17 of the living body measuring element 12, as shown in Figure 2.

The light that is emitted from the light source 11 and reaches the living body measuring optical element 12, goes to the groovy portion 17 provided in the living body measuring optical element 12. Then, the light from the groovy portion 17 strikes the light guide 14. After being refracted or scattered by the light guide and the light again strikes the living body measuring optical element 12.

The incident angle of the light incident on the groovy portion 17 can be determined based on the shape of the groovy portion 17, refractory indexes and absorption coefficients of the living body measuring optical element 12 and the light guide, or the incident angle with respect to the groovy portion 17.

In the biological information measuring apparatus of the present invention, it is preferable that light reaches the light detector 16 to the most extent when a living tissue is measured. Therefore, it is preferable that the shape of grooves, incident angle, and refractive index and absorption coefficient of the light guide are set in accordance with the refractive index of the living tissue.

Accordingly, if the refractive index of the light guide is larger than that of the air and lower than that of the living body measuring optical element 12, the calibration mentioned above is effectively performed. The refractive index of the light guide is preferably closer to that of the living body, and particularly preferably about 1.2 to 1.4.

The light which has reached the living body measuring optical element 12 passes through the light guide 14 in contact with the groovy portion 17 in this state, and returns again to the living body measuring optical element 12 and reaches the light detector 16.

The signal detected at this point is stored in memory means 22 as a calibration signal.

A calibration signal value obtained where the light source 11 and the light detector 16 are in normal conditions is used as a reference value. The reference value is stored in the memory means 22 (in the memory means 22) in advance. The calibration signal value stored by the calculation section 21 in the memory means 22 is then compared to the reference value. If the calibration signal value stored in the memory means 22 fails to fall within the range of the reference value, it is considered that the light source 11, the light detector 16 or the living body measuring optical element 12 is faulty. In this case, the display section 23 displays a message indicating that the light source 11, the light detector 16 or the living body measuring optical element 12 is faulty or informs the message by voice. When the display section 23 gives such a notice by display or voice, it is preferable that the light source 11, the light detector 16 or the living body measuring optical element 12 be replaced with new one.

When a calibration signal value is normal, the light guide 14 is removed from the living body measuring optical element 12 and the living body measuring optical element 12 is brought into contact with a living tissue to measure biological information (not shown in the figure).

The living tissue is not particularly limited; however, mouth lip, forearms, fingers and ears are preferably employed.

The light is emitted from the light source and reaches the living body measuring optical element 12, and thereafter, passes through the living tissue in contact with the groovy portion 17, and reaches the light detector.

This signal, unlike the signal passed through the light guide 14, contains biological information since it passed through the living tissue.

In general, this measurement value is greatly affected when the light intensity of the light source 11 and the sensitivity of the light detector 17 change with age.

To correct such effect, the calibration signal value measured as described above is used.

For example, in the calculation section 21, a measurement value containing biological information is divided by the calibration signal value. Through calculation using the resultant quotient, biological information is obtained.

By dividing a measurement value containing biological information by the calibration signal value in the calculation section 21 (the effect of), secular change and temperature change in the light source and the light detector can be suppressed. As a result, measurement can be attained successfully even if the intensity of the light source and the sensitivity of the light detector change.

As described in the foregoing, according to this embodiment, by using a reference element having a light guide capable of guiding light applied by a biological information measuring optical element so that the light can be returned to the living body measuring optical element in the state where the light guide is arranged in contact with the living body measuring optical element and by using a biological information measuring apparatus equipped with the light guide, the sensitivity of the light source, living body measuring optical element and light detector can be checked, thus a measurement error due to temperature change and secular change can be corrected.

Either of the operations: measurement performed by bringing the light guide 14 into contact with the living body measuring optical element 12; and measurement performed by bringing the living body surface into contact with the living body measuring optical element 12, may be performed first.

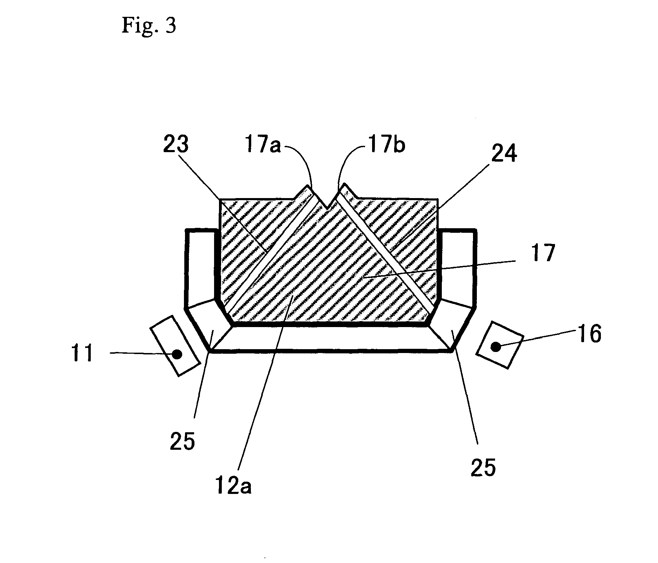

In this embodiment, it was described that the living body measuring optical element 12 is as shown in Figure 1. However, the element is not limited to this type and a living body optical element 12a shown in Figure 3 may be used. The living body optical element 12a has a groovy portion 17, which is formed of a light emitting surface 17a and a light incident surface 17b, an optical fiber 23 for guiding the light emitted from the light source 11 to the groovy portion 17a, and an optical fiber 24 for guiding the incident light on the light incident surface 17b to the light detector 16. The living body measuring optical element 12a is used to measure biological information by pressing the groovy portion 17 of the living body measuring optical element 12a against the surface of the living body and detecting light passing through the portion of the living body trapped by the groovy portion 17. If a living body measuring optical element 12a of this configuration is used in place of the living body measuring optical element 12, the same effect as that of this embodiment can be achieved.

### Industrial Applicability

The biological information measuring apparatus, a reference element and the method of using the biological information measuring apparatus according to the present invention have the following advantages: the sensitivity of the light source, the living body measuring optical element and the light detector can be checked and measurement errors caused by temperature change and secular change can be corrected. Therefore, they are useful for non-invasive measurement of glucose, cholesterol, urea, triglyceride and so forth in body fluids based on optical measurement of the living tissues. Consequently, the present invention is useful for measuring body fluid components in medical applications.

## Claims

1. A biological information measuring apparatus comprising:
a light source;
a living body measuring optical element of applying light emitted from said light source to a living body and receiving light returning from said living body;
a light detector of detecting said light received by said living body measuring optical element; and
a reference light guide capable of guiding the light applied by said living body measuring optical element so that the light can be returned to said living body measuring optical element, in a state of being arranged in contact with said living body measuring optical element.

2. The biological information measuring apparatus according to claim 1, further comprising a calculation section of providing biological information of said living body through calculation based on the light returned from said living body and detected by said light detector,
wherein the calculation section detects that at least one of said light source, said living body measuring optical element and said light detector abnormally functions based on the light detected by said light detector in a state where said light guide is arranged in contact with said living body measuring optical element.

3. The biological information measuring apparatus according to claim 1, further comprising a calculation section of providing biological information of said living body through calculation based on the light returning from said living body and detected by said light detector,
wherein the calculation section corrects said biological information based on the light detected by said light detector in a state where said light guide is arranged in contact with said living body measuring optical element.

4. The biological information measuring apparatus according to claim 1, wherein a concave-convex portion is formed on a part of the surface of said living body measuring optical element, and the part of said light guide which is to be in contact with said living body measuring optical element is deformable.

5. The biological information measuring apparatus according to claim 1, wherein said light guide is formed of a material having a refractive index higher than that of the air and lower than that of said living body measuring optical element.

6. The biological information measuring apparatus according to claim 1, wherein said light guide is a scattering body.

7. The biological information measuring apparatus according to claim 4, wherein said light guide is an elastic substance.

8. The biological information measuring apparatus according to claim 7 , wherein said light guide has an elasticity modulus of 1 to 10 MPa.

9. A reference element for use in a biological information measuring apparatus comprising:
a light source;
a living body measuring optical element of applying light emitted from said light source to a living body and receiving light returning from said living body; and
a light detector of detecting the light received by said living body measuring optical element,
the reference element comprising:
a light guide capable of guiding the light applied by said living body measuring optical element so that it returns to said living body measuring optical element in a state of being arranged in contact with said living body measuring optical element.

10. The reference element according to claim 9, further comprising a cover of covering a portion of said light guide other than the portion which is in contact with said living body measuring optical element.

11. The reference element according to claim 9, wherein a part of said light guide which is in contact with said living body measuring optical element is deformable.

12. The reference element according to claim 9, wherein said light guide is formed of a material having a refractive index higher than that of the air and lower than that of said living body measuring optical element.

13. The reference element according to claim 9, wherein said light guide is a scattering body.

14. The reference element according to claim 11, wherein said light guide is an elastic substance.

15. The reference element according to claim 14, wherein said light guide has an elasticity modulus of 1 to 10 MPa.

16. A method of using a biological information measuring apparatus using the biological information measuring apparatus according to claim 1, comprising:
a biological information measuring step of measuring biological information based on the light detected by said light detector in a state where said living body measuring optical element is in contact with a target living body to be measured; and
an abnormality-correcting step of detecting or correcting an abnormality based on the light detected by said light detector in a state that said reference light guide is in contact with said living body measuring optical element.
